# EUROPEAN PATENT SPECIFICATION

(11) **EP 4 019 934 B1**
(45) Date of publication and mention of the grant of the patent: **16.10.2024**
(21) Application number: 21217290.2
(22) Date of filing: 23.12.2021
(51) Int. Cl.: G01N 15/06, G01N 35/00, G01N 23/00

(54) **PORTABLE ON-LINE IN-SITU BETA-RAY MONITOR AND MONITORING METHOD**
TRAGBARER ONLINE-IN-SITU-BETASTRAHLMONITOR UND ÜBERWACHUNGSVERFAHREN
MONITEUR EN LIGNE PORTABLE DE RAYONS BETA IN SITU ET PROCÉDÉ DE SURVEILLANCE

(30) Priority: 23.12.2020 CN 202011544330
(43) Date of publication of application: 29.06.2022
(73) Proprietor: Tianjin Zwinsoft Technology Co., Ltd., Tianjin 300000 (CN)
(72) Inventor: SHEN, Qi, Binhai New District, 300000 (CN); CAO, Lifeng, Binhai New District,, 300000 (CN); WEI, Jingyu, Binhai New District, 300000 (CN); WANG, Yuansheng, Binhai New District, 300000 (CN); CHEN, Tao, Binhai New District, 300000 (CN)
(74) Representative: Gill Jennings & Every LLP

(56) References cited:
- CN-A- 109 254 316
- CN-U- 206 920 288
- CN-U- 210 953 726
- CN-U- 210 953 727
- CN-Y- 2 548 153
- KR-A- 20160 103 766

## Description

### TECHNICAL FIELD

The present disclosure relates to the technical field of detection of particulate matters in gas, and in particular, to a portable on-line in-situ β-ray monitor and a monitoring method.

### BACKGROUND ART

With wide application of technologies of wet electric dust removal, low-temperature electric dust removal, and the like in ultra-low emission transformation of coal-fired power plants, iron and steel, coking, and other enterprises, most of concentrations of smoke dust at a discharge port have reached below 10 mg/m³, which puts forward higher requirements for the detection accuracy of particulate matters in fume. The existing β-ray-based on-line monitor determines the concentration of ambient dust by measuring the adsorbed radiation quantity of the ambient dust in the atmosphere by using a β-ray absorption principle. The more rays are adsorbed, the higher the concentration of the ambient dust is; and on the contrary, the less rays are adsorbed, the lower the concentration of the ambient dust is. An ambient dust concentration monitor is specially designed for monitoring the ambient dust in ambient air. It is mainly used for the measurement of the concentration of the particulate matters in the fume by an absorption method in the fields of atmosphere quality monitoring networks, mobile monitoring stations, long-term background environment researches, industrial and mining enterprises, scientific research institutes, and the like. However, the existing monitor is not precise enough during filter tape feeding and collecting, and filter tape pressing and the filter tape feeding are prone to errors, which results in inaccurate collected data and large deviation of monitoring results.

CN 206 920 288 U discloses an automatic device of survey binary channels particulate matter volume concentration, particulate matter separation sampling module is connected with appearance air humidity degree control module, and appearance air humidity degree control module's gas outlet is connected twin -channel sampling and is detected the module, twin -channel sampling detects two tunnel gas outlets of module and is connected with two way constant -current regulation modules respectively, the gas outlet of two way constant -current regulation modules is connected with the sampling pump through the three -way valve. CN 206 920 288 U discloses but the concentration by mass of PM 10 and 2.5 particulate matters of PM in the device simultaneous determination atmospheric environment has simple structure, low, long, the quick accuracy of data of cycle of operation of maintenance cost, really realizes online automatic continuous monitor. CN 206 920 288 U discloses a beta ray detector and a beta radiation source only needing really to have been realized by the device, both can accomplish the continuous online sampling process of binary channels, have simple structure, low, long, the quick accuracy of data of cycle of operation of maintenance cost, have reduced instrument cost and later maintenance cost, really realize online automatic continuous monitor. CN 206 920 288 U discloses the preamble of claim 1.

CN 210 953 726 U provides a portable atmospheric particulate concentration monitoring mechanism, which comprises a sampling rod and a monitoring host, and the monitoring host comprises a box body; the driving device comprises an air port movement unit and a paper tape movement unit; a sealing device; a flow rate control device; a detection device; wherein the paper tape moving unit comprises a paper tape feeding unit and a paper tape discharging unit; the device comprises a base, a paper tape, a first tape reel, a second tape reel in transmission connection with the first tape reel and a supporting wheel, the paper tape is wound on the first tape reel through the second tape reel, the supporting wheel and the detection device, the distance between the first tape reel and the second tape reel ranges from 5 cm to 15 cm, and the surface of the detection device is provided with an arc surface. The portable atmospheric particulate concentration monitoring mechanism provided by CN 210 953 726 U is simple and compact in structure, convenient to carry and small in load.

CN 210 953 727 U provides an atmospheric particulate concentration monitoring mechanism, which comprises a sampling rod and a monitoring host, and the monitoring host comprises a box body; the driving device comprises an air port movement unit and a paper tape movement unit; a sealing device; a flow rate control device; a detection device; wherein the gas port movement unit comprises a first motor, an eccentric shaft and a lifting unit communicated with the sampling rod; wherein one end of the eccentric shaft is connected with a rotating shaft of the first motor, the other end of the eccentric shaft is connected with the lifting unit, and the first motor drives an air port of the lifting unit to be in contact with and separated from a paper tape of the paper tape movement unit through the eccentric shaft. The atmospheric particulate concentration monitoring mechanism provided by CN 210 953 727 U has the advantages of simple and compact structure, no abnormal sound, small load and convenience in measurement.

KR 2016 0103766 A relates to a fine dust automatic measuring device capable of simultaneously collecting heavy metal samples. The fine dust automatic measuring device can automatically measure a concentration of fine dust in air by using beta rays, and can simultaneously extract samples for analyzing components such as heavy metals. The fine dust automatic measuring device comprises: a body including an inlet disposed in the upper side thereof to feed a particular flow rate of air including fine dust, including an outlet disposed in the lower side thereof to discharge air having fine dust removed therefrom, and including a flow path for flowing air; a collecting filter sheet disposed in the flow path, and collecting fine dust included in flowing air; a beta ray irradiating unit disposed in the upper side of the collecting filter sheet in the flow path to be separated at particular distance therefrom, and irradiating beta rays toward the collecting filter sheet; and a beta ray detecting unit dispose din the lower side of the collecting filter sheet in the flow path, and detecting beta rays penetrating through the collecting filter sheet.

### SUMMARY

In view of the shortcomings in the prior art, an objective of the present disclosure is to provide a portable on-line in-situ β-ray monitor and a monitoring method as set out in the appended set of claims.

In order to achieve the above objective, the present disclosure provides the following technical solution:

A portable on-line in-situ β-ray monitor and a monitoring method include a detection mechanism and a control mechanism electrically connected to each other. The detection mechanism includes a filter tape feed device, a filter tape pressing device, a detector, and a gas guide device. The gas guide device is arranged in the filter tape pressing device in a penetrating manner to guide a gas to be tested on the test filter tape. The filter tape pressing device is arranged right above the detector and is configured for pressing the test filter tape. The detector is configured for counting. The control mechanism includes a first detector and a second detector. The filter tape pressing device is connected to the first detector. The filter tape feed device is connected to the second detector. The filter tape feed device is configured for storing and moving test filter tape. The first detector is configured for detecting a down-pressing situation of the filter tape pressing device and the second detector is configured for detecting a filter tape feeding in-place situation.

The filter tape pressing device includes a head pressing block and a filter tape pressing holder. A head pressing disc is fixedly arranged outside the head pressing block in a sleeving manner. An upper end of the head pressing disc is fixedly connected to the filter tape pressing holder through a spring.

A lower end of the head pressing block is connected to a head pressing membrane. An upper end of the pressing head block is connected to a head pressing gas pipe. The bottom of the pressing head gas pipe is fixed to an upper end of the filter tape pressing holder through a head pressing seat.

In the present disclosure, optionally, a gas guide chamber is formed in the head pressing block. An end cover is fixed to the bottom of the head pressing block and forms a gas outlet at the bottom of the head pressing block. The gas guide device is fixed to the inner side of the end cover. The gas guide device and the gas outlet are arranged coaxially.

In the present disclosure, optionally, the detector includes a detecting frame. A head pressing plate is fixed to the top of the detecting frame. A detecting hole is formed at a position, fixedly position where the head pressing plate is fixedly connected to the detecting frame. A detector is fixed coaxially to a lower end of the detecting hole.

In the present disclosure, optionally, the control mechanism includes a main control configured to realize overall control of the monitor. The main control panel is connected to a drive group through a relay and the main control panel controls the gas pump to work through the relay. The drive group comprises a filter tape drive motor, a flow rate control motor, and a filter tape pressing drive motor. The filter tape feed device comprises a primary filter tape belt pulley, a secondary filter tape belt pulley, and two limiting wheels, the primary filter tape belt pulley is connected to a filter tape drive motor; and the filter tape drive motor drives the primary filter tape belt pulley to rotate to feed filter tape. The flow rate control motor controls the overall gas inlet amount of the monitor. The main control panel controls the flow rate control motor to work through the relay so as to drive a regulating valve to work. The filter tape pressing drive motor is fixed to a mounting plate through a filter tape pressing motor frame and the filter tape pressing drive motor works so that the pressing block of the filter tape pressing device performs an operation of pressing the filter tape or lifting up the filter tape. An output shaft of the filter tape pressing drive motor is connected to a cam, a side of the cam is connected to the head pressing disc and the cam rotates under the driving of the filter tape pressing drive motor.

In the present disclosure, optionally, the output shaft of the filter tape pressing drive motor is also fixedly connected to the cam through the first detector.

In the present disclosure, optionally, a mounting plate is fixed between the detecting mechanism and the control mechanism. A connecting hole is formed in the position, corresponding to the gas outlet of the detecting frame, of the mounting plate. The connecting hole is connected to a gas guide pipe. The other end of the gas guide pipe is connected to a flow regulating device. The flow regulating device includes a flowmeter. The flowmeter is connected between the connecting hole and the regulating valve through the gas guide pipe, and the other end of the regulating valve is connected to a gas pump.

In the present disclosure, optionally, the mounting plate is fixedly arranged in the middle of a box body. A collecting mechanism is fixedly arranged on the box body.

A portable on-line in-situ β-ray monitoring method includes the following steps:
S1: lifting up a filter tape pressing device, moving test filter tape in place by a filter tape feed device, and performing initial counting by the detector;
S2: after the counting is completed, pressing down the filter tape pressing device, introducing a gas to be tested through a gas guide device, and performing collecting;
S3: after collecting is completed, lifting up the filter tape pressing device, and performing collecting counting by a detector; and
S4: after the counting is completed, moving new test filter tape in place by using the filter tape feed device, and performing next round of collecting.

Compared with the prior art, the present disclosure has the following beneficial effects.

According to the device of the present disclosure, ambient dust collection and detection are set in the same channel, which avoids an error caused by feeding filter tape for a plurality of times. Meanwhile, the filter tape pressing device is connected to the first detector. The head pressing block is also connected to the filter tape pressing drive motor through the cam, so that the head pressing block can be pressed down or lifted up very well, and the sealing property during filter tape pressing and smooth filter tape feeding after lifting up the head pressing block can be ensured. Meanwhile, the flow regulating device is also arranged, and constant flow sampling is realized by the control mechanism, which further ensures the measurement accuracy. In addition, the mounting plate is arranged reasonably, and is mounted surface by surface according to functions, which ensures the performance, meanwhile, saves the space, reduces the size, and is portable.

### BRIEF DESCRIPTION OF THE DRAWINGS

FIG. 1 is a control block diagram of a portable on-line in-situ β-ray monitor according to the present disclosure.
FIG. 2 is an installation diagram of a front surface of a mounting plate of the portable on-line in-situ β-ray monitor according to the present disclosure.
FIG. 3 is an installation diagram of a back surface of the mounting plate of the portable on-line in-situ β-ray monitor according to the present disclosure.
FIG. 4 is a vertical view of the mounting plate of the portable on-line in-situ β-ray monitor according to the present disclosure.
FIG. 5 is a sectional view of a filter tape pressing device and a detector of the portable on-line in-situ β-ray monitor at 4A-A according to the present disclosure.
FIG. 6 is a schematic diagram of a connecting structure of the filter tape pressing device and a filter tape pressing drive motor of the portable on-line in-situ β-ray monitor according to the present disclosure.
FIG. 7 is a structural schematic diagram of a gas guide device of the portable on-line in-situ β-ray monitor according to the present disclosure.

Reference signs in the drawings: 1-filter tape feed device, 11-primary filter tape belt pulley, 12-secondary filter tape belt pulley, 120-secondary filter tape belt pulley mounting frame, and 13-two limiting wheels;
2-filter tape pressing device, 21-head pressing block, 22-filter tape pressing holder, 23-head pressing disc, 24-head pressing membrane, 25-head pressing gas pipe, 26-gas guide chamber, 27-end cover, 28-gas outlet, and 29-head pressing plate;
3-detector, 31-detection frame, 32-detection hole, and 33-detector;
4-gas guide device, 41-first gas guide component, 42-second gas guide component, 43-diversion port, and 44-placement chamber;
5-first detector, 51-head pressing limiting piece, and 52-first limit switch;
6-second detector, 61-second limiting piece, and 62-second limit switch;
7-main control panel, 71-relay, 72-filter tape drive motor, 73-flow rate control motor, 74-filter tape pressing drive motor, 741-filter tape pressing motor frame, 742-cam, and 75-power supply;
81-flowmeter, 82-regulating valve, and 83-gas pump;
90-temperature and humidity collector, 91-wind speed collector, and 92-wind direction collector;
100-mounting plate, 101-connecting hole, and 102-display fixing frame.

### DETAILED DESCRIPTION OF THE EMBODIMENTS

Technical solutions in the embodiments of the present disclosure will be clearly and completely described herein below with reference to the drawings in the embodiments of the present disclosure. Apparently, the described embodiments are merely part rather than all of the embodiments of the present disclosure. On the basis of the embodiments of the present disclosure, all other embodiments obtained by those of ordinary skill in the art without creative work fall within the scope of protection of the present disclosure.

It is to be noted that when a component is described as being "fixed" to another component, it may be directly on the other component, or there may be a component therebetween. When a component is regarded as being "connected" to the other component, it may be directly connected to the other component, or there may be a component therebetween. When a component is regarded as being "arranged" on the other component, it may be directly arranged on the other component, or there may be a component therebetween. The terms "vertical", "horizontal", "left", "right", and similar expressions used herein are merely for the purpose of illustration.

Unless otherwise defined, all technical and scientific terms used herein shall have the same meanings as commonly understood by those skilled in the art to which this present disclosure belongs. The terms used herein in the specification of the present disclosure are only used to describe specific embodiments, and are not intended to limit the present disclosure. The term "and/or" used herein includes any and all combinations of one or more of the associated listed items.

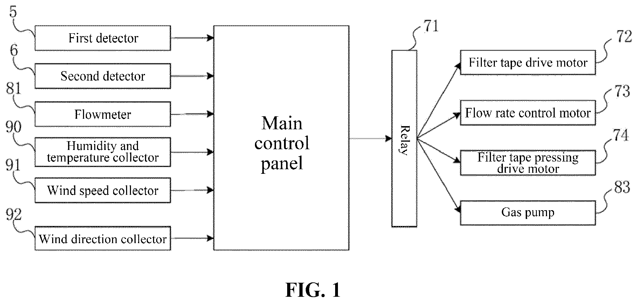

Referring to FIG. 1 to FIG. 4 at the same time, a preferred implementation manner of the present disclosure provides a portable on-line in-situ β-ray monitor, which mainly determines the concentration of ambient dust by measuring the adsorbed radiation quantity of the ambient dust in the atmosphere by using a β-ray absorption principle. Ambient dust collection and detection are set in the same channel, which avoids an error caused by feeding filter tape for a plurality of times. An in-place detection device is arranged on each of a filter tape feed device 1 and a filter tape pressing device 2, so as to ensure the accuracy of filter tape feeding and filter tape pressing and avoid an error. A flow regulating device is also arranged to perform constant flow sampling, which ensures the measurement accuracy. The monitor mainly includes a detecting mechanism and a control mechanism electrically connected to each other. The control mechanism controls the detecting mechanism to detect. The detection mechanism and the control mechanism are respectively fixed to a front surface and a back surface of a mounting plate 100. The detecting mechanism includes the filter tape feed device 1, the filter tape pressing device 2, a detector 3, and a gas guide device 4. The gas guide device 4 is arranged in the filter tape pressing device 2 in a penetrating manner. The filter tape pressing device 2 is arranged right above the detector 3. The control mechanism includes a first detector 5 and a second detector 6. The filter tape pressing device 2 is connected to the first detector 5. The filter tape feed device 1 is connected to the second detector 6.

Specifically, the filter tape feed device 1 is fixed below the mounting plate 100. The filter tape feed device 1 is used for storing and moving test filter tape. A second detector 6 connected to the filter tape feed device 1 is mainly used for detecting a filter tape feeding in-place situation. The filter tape pressing device 2 is used for pressing the test filter tape. The first detector 5 is used for detecting a down-pressing situation of the filter tape pressing device 2. The gas guide device 4 is arranged in the filter tape pressing device 2 to guide a gas to be tested on the test filter tape. The detector 3 is fixed right below the filter tape pressing device 2, and is used for collecting particulate matters in the ambient dust on the test filter tape. The whole process is controlled by a control device to automatically realize filter tape feeding, filter tape pressing, gas introducing, and collecting, and the concentration of ambient dust in the atmosphere is obtained through the processing of the control mechanism. The filter tape pressing device 2 and the detector 3 are arranged coaxially, so that the collecting can be completed by feeding filter tape once. In addition, the first detector 5 and the second detector 6 detect the situations of the filter tape pressing device 2 and the filter tape feed device 1 in real time, and sends the situations to the control mechanism, which avoids errors caused by excessive or too little filter tape feeding or not pressing down or lifting up the filter tape pressing device 2 during the process. The whole process is controllable and can be performed smoothly. Meanwhile, the test filter tape is tightly pressed through the filter tape pressing device 2, which avoids inaccuracy of the collected data caused by the diffusion of the gas to be tested.

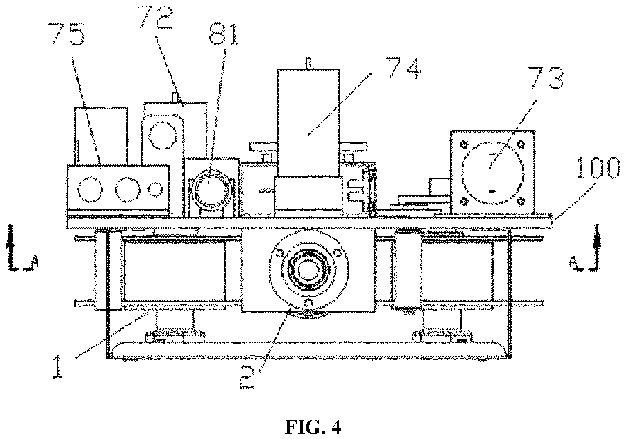

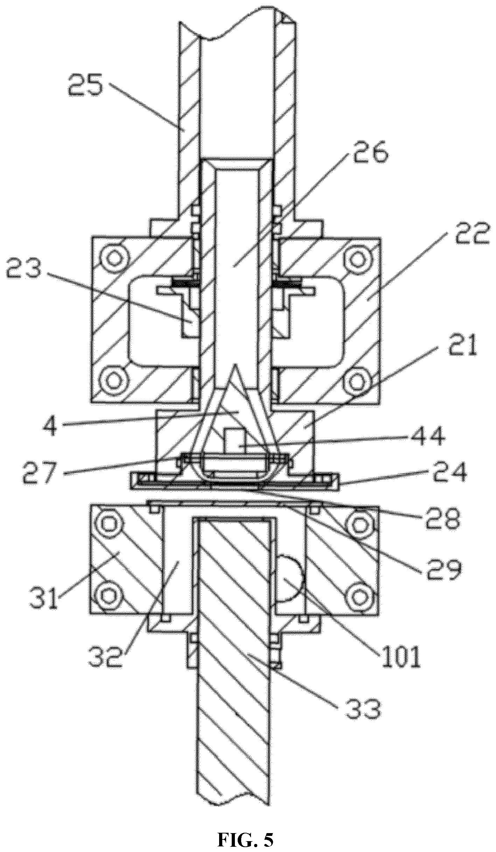

Referring to FIG. 4 and FIG. 5, in the present embodiment, the filter tape pressing device 2 includes a head pressing block 21 and a filter tape pressing holder 22. The filter tape pressing holder 22 is fixed to the mounting plate 100. A head pressing disc 23 is arranged outside the head pressing block 21 in a sleeving manner. An upper end of the head pressing disc 23 is fixedly connected to the filter tape pressing holder 22 through a spring. A lower end of the head pressing block 21 is connected to a head pressing membrane 24. An upper end of the head pressing block is connected to a head pressing gas pipe 25. The bottom of the pressing head gas pipe 25 is fixed to an upper end of the filter tape pressing holder 22 through a head pressing seat. A gas guide chamber 26 is formed in the head pressing block 21. An end cover 27 is fixed to the position, where a gas outlet 28 is formed, at the bottom of the head pressing block 21. The gas guide device 4 is fixed to the inner side of the end cover 27. The gas guide device 4 and the gas outlet 28 are arranged coaxially.

Specifically, the filter tape pressing holder 22 is fixed to the middle of the mounting plate 100. The head pressing block 21 is arranged in the filter tape pressing holder 22 in a penetrating manner. The head pressing disc 23 is fixed to the outer side of the part, located in the filter tape pressing holder 22, of the head pressing block 21. An upper end of the head pressing disc 23 is fixedly connected to the filter tape pressing holder 22 through the spring, so that the head pressing block 21 is driven to move up and down for a certain distance by the head pressing disc 23 under the driving of an external force, thereby facilitating the movement of the test filter tape below the head pressing block 21. A gas guide chamber 26 is formed in the head pressing block 21. The gas to be tested enters the gas guide chamber 26 through the pressing head gas pipe 25 connected above, comes out from the gas outlet 28 after passing through the gas guide device 4 in the gas guide chamber 26, reaches filter tape to be tested below, and passes through the filter tape to be tested to remain particulate matters on the filter tape to be tested.

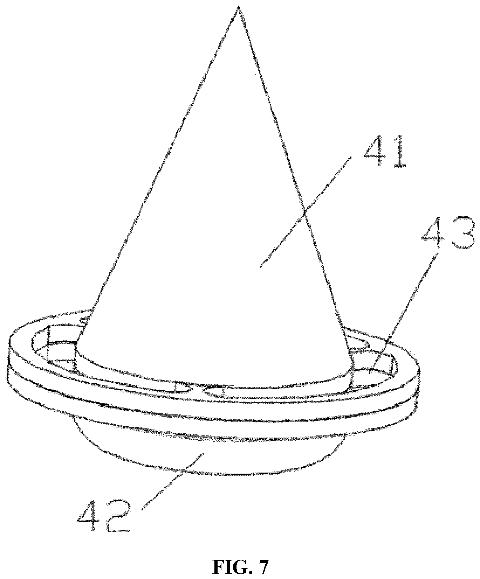

Referring to FIG. 7, further, the gas guide device 4 includes a first gas guide component 41 and a second gas guide component 42. The first gas guide component 41 is in an inverted conical shape, and the second gas guide component 42 is in a semicircular shape. A connecting edge is fixed to the outer sides of the first gas guide component 41 and the second gas guide component 42. A diversion port 43 is formed in the inner side of the connecting edge, and is used for the circulating of the gas to be tested. Such arrangement has strong guidance to the gas to be tested, so that all gas can be collected to the gas outlet 28. A placement chamber 44 is formed in the first gas guide component 41 and the second gas guide component 42. A β-ray emitter is placed in the placement chamber 44. The β-ray emitter directly faces the gas outlet 28 below. After the collecting of the filter tape to be tested is completed, a beam emitted by the β-ray emitter is received by the detector 3 below after passing through the filter tape to be tested, and is transmitted to the control mechanism, so as to complete the collecting of data.

In the present embodiment, the detector 3 includes a detecting frame 31. The detecting frame 31 is fixed to the mounting plate 100. A head pressing plate 29 is fixed to the top of the detecting frame 31. A detecting hole 32 is formed in a position where the head pressing plate 29 is fixedly connected to the detecting frame 31. A detector 33 is fixed coaxially to a lower end of the detecting hole 32.

Specifically, the filter tape feed device 1 includes a primary filter tape belt pulley 11, a secondary filter tape belt pulley 12, and two limiting wheels 13. The primary filter tape belt pulley 11 is connected to a filter tape drive motor 72. The limiting wheel 13 close to the primary filter tape belt pulley 11 is connected to the second detector 6. The second detector 6 includes a second limiting piece 61 and a second limit switch 62. The second limiting piece 61 and the second limit switch 62 are fixedly arranged on a back surface of the mounting plate 100, correspond to the limiting wheels 13, and are used for detecting a rotating angle of the limiting wheels 13 so as to confirm a filter tape feeding situation. The primary filter tape belt pulley 11 and the secondary filter tape belt pulley 12 are respectively arranged on two sides of the detector 3. The filter tape to be tested is connected from the primary filter tape belt pulley 11 into the secondary filter tape belt pulley 12 after passing through the first limiting wheel 13, the head pressing plate 29, and the second limiting wheel 13. The primary filter tape belt pulley 11 rotates under the driving of the filter tape drive motor 72, so as to drive the filter tape to be tested to move. After the moving is completed, the head pressing block 21 is pressed down to be in contact with the head pressing plate 29, so as to fix the filter tape to be tested, and then collecting is performed. After the collecting is performed for 20 to 50 minutes, the detector 33 performs counting to complete the collecting of atmosphere particulate matter data, and the atmosphere particulate matter data is transmitted to the control mechanism.

In the present embodiment, the control mechanism includes a main control panel 7. The main control panel 7 realizes overall control of the system. The main control panel 7 is connected to a drive group through a relay 71. The drive group includes the filter tape drive motor 72, a flow rate control motor 73, and a filter tape pressing drive motor 74. The filter tape pressing drive motor 74 is fixed to the mounting plate 100 through a filter tape pressing motor frame 741, and corresponds to the head pressing block 21.

Specifically, both the first detector 5 and the second detector 6 are connected to the main control panel 7. The collected signal is transmitted to the main control panel 7. The main control panel 7 determines whether to perform a next operation instruction according to an input signal. When the next instruction needs to be performed, the main control panel 7 sends a signal to the relay 71, so that the corresponding relay 71 is sucked to enable the drive group connected to the relay 71 to work, i.e., the filter tape drive motor 72 drives the primary filter tape belt pulley 11 to rotate to feed filter tape, or the flow rate control motor 73 control the overall gas inlet amount of the system, or the filter tape pressing drive motor 74 works, so that the filter tape pressing device 2 performs an operation of pressing the filter tape or lifting up the filter tape.

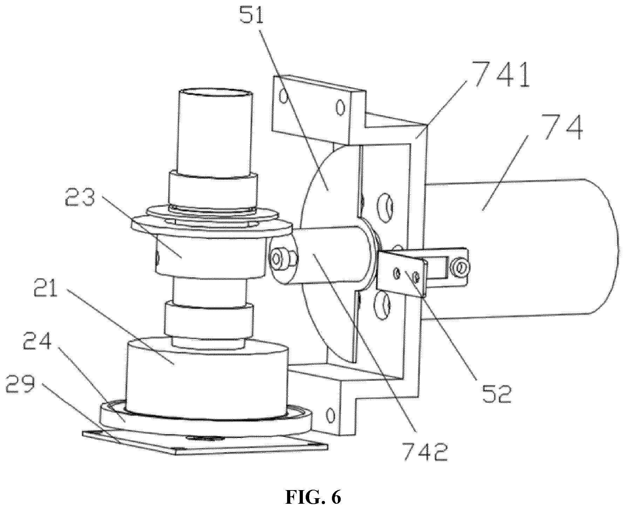

Referring to FIG. 6, in the present embodiment, an output shaft of the filter tape pressing drive motor 74 is fixedly to a cam 742 through the first detector 5.

Specifically, an output shaft of the filter tape pressing drive motor 74 is connected to the cam 742. A side of the cam 742 is connected to the head pressing disc 23. The cam 742 rotates under the driving of the filter tape pressing drive motor 74. When a convex edge of the cam rotates to be in contact with the head pressing disc 23, the head pressing disc 23 is jacked up, so that the head pressing block 21 moves upward to be separated from the test filter tape, thereby facilitating the movement of the test filter tape. When the cam 742 rotates to the other side, the head pressing disc 23 moves downward under the action of the spring, so that the head pressing block 21 is pressed down to be in contact with the test filter tape, thereby performing collecting work. The first detector 5 includes a first head pressing limiting piece 51, and a first limit switch 52 is fixed next to the filter tape pressing drive motor 74. The first head pressing limiting piece 51 is matched with the first limit switch 52 to detect an output rotating angle of the filter tape pressing drive motor 74, which ensures that a rotating direction of the cam 742 in place, thereby ensuring that the head pressing block 21 is pressed down or lifted up in place.

In the present embodiment, a connecting hole 101 is formed in the position, corresponding to the gas outlet of the detecting frame 31, of the mounting plate 100. The connecting hole 101 is connected to a gas guide pipe. The other end of the gas guide pipe is connected to a flow regulating device. The flow regulating device includes a flowmeter 81. The flowmeter 81 is connected between the connecting hole 101 and the regulating valve 82 through the gas guide pipe, and the other end of the regulating valve 82 is connected to a gas pump 83.

Specifically, the gas pump 83, the flow rate control motor 73, and the flowmeter 81 are all connected to the main control panel 7. During collecting, the main control panel 7 controls the gas pump 83 to work through the relay 71, so as to pump the gas to be tested into the monitor. The gas is exhausted through the gas pump 83 after passing through the head pressing gas pipe 25, the head pressing block 21, the gas outlet 28, the filter tape to be tested, the head pressing plate 29, the detecting frame 31, the connecting hole 101, the gas guide pipe, the flowmeter 81, and the regulating valve 82 in sequence. The flowmeter 81 detects gas flow, and sends a detected signal to the main control panel 7. When the gas flow is greater than or equal to a set value, the main control panel 7 controls the flow rate control motor 73 to work through the relay 71, so as to drive the regulating valve 82 to work to control the gas flow to be kept in a constant interval, thereby ensuring the measurement accuracy.

In the present implementation mode, the mounting plate 100 is fixedly arranged in the middle of a box body, and a collecting mechanism is fixedly arranged on the box body.

Specifically, the mounting plate 100 is fixed to the middle of a box body. The detecting mechanism and the control mechanism are respectively fixed to a front surface and a back surface of the mounting plate 100. The detecting mechanism is on the front surface of the mounting plate 100, and the control mechanism is on the back surface of the mounting plate 100. Output ends of the filter tape drive motor 72 and the filter tape pressing drive motor 74 penetrate through the mounting plate 100 to connect the corresponding primary filter tape belt pulley 11 and the cam 742. A display fixing frame 102 is also fixed to the outer side of the filter tape pressing device 2 on the front surface of the mounting plate 100, and is mainly used for fixedly mounting a display panel. The display panel is connected to the main control panel 7, and is used for displaying collected data and the like, which is convenient for personnel to view on site. The collecting mechanism includes a temperature and humidity collector 90, a wind speed collector 91, and a wind direction collector 92. The collecting mechanism is connected to the main control panel 7, and sends to the collected data to the main control panel 7.

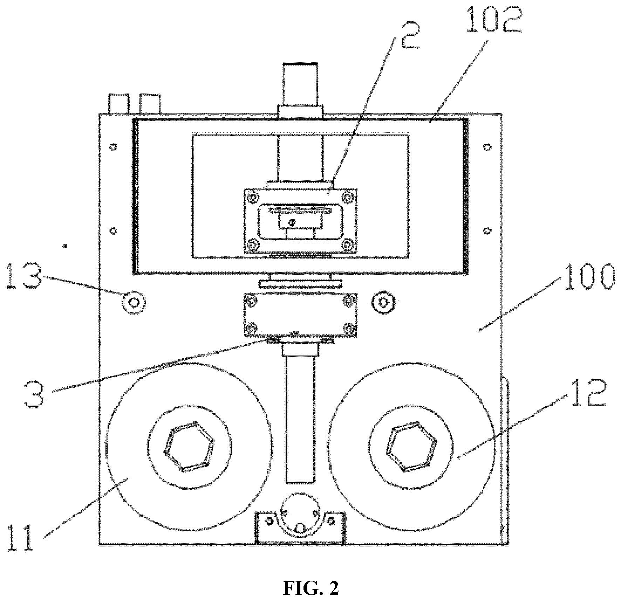

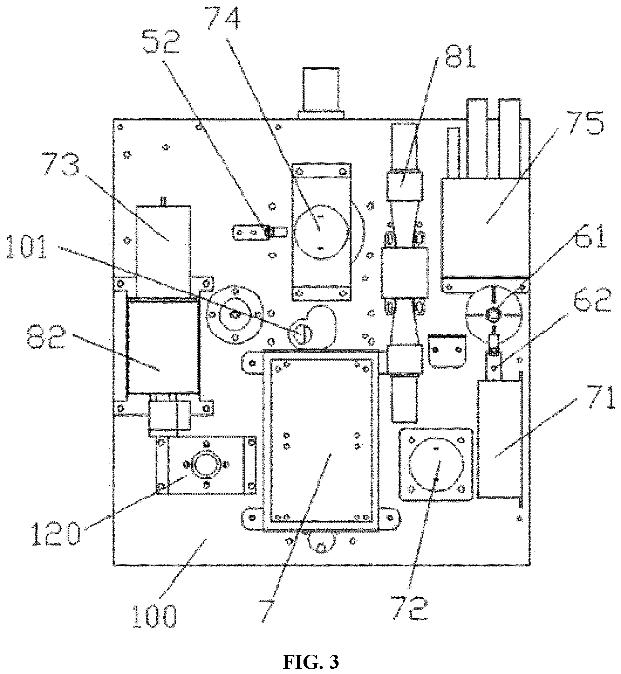

As shown in FIG. 2 and FIG. 3, further, in order to fully utilize the space and reduce the overall size of a detecting instrument, the display panel, the detector 3, and the filter tape feed device 1 are mounted on the front surface of the mounting plate 100 in sequence from top to bottom. The filter tape pressing device 2 is fixed below the display panel. The flow rate control motor 73, the filter tape pressing drive motor 74, the flowmeter 81, and a power supply 75 are fixed to the back surface of the mounting plate 100 in sequence from top to bottom and from left to right. The regulating valve 82 is connected below the flow rate control motor 73. The connecting hole 101 is formed below the filter tape pressing drive motor 74. The second detector 6 is fixed below the power supply 75. A secondary filter tape belt pulley mounting frame 120 is fixed below the regulating valve 82. The main control panel 7 is fixed below the connecting hole 101. The filter tape drive motor 72 is fixed to the right side of the main control panel 7. The relay 71 is fixed to the right side of the filter tape drive motor 72.

Another preferred implementation manner of the present disclosure provides a monitoring method of a portable on-line in-situ β-ray monitor, including the following steps:
S1: the filter tape pressing device 2 is lifted up, test filter tape is moved in place by the filter tape feed device 1, and initial counting is performed.
S2: after the counting is completed, the filter tape pressing device 2 is pressed down, a gas to be tested is introduced through the gas guide device 4, and collecting is performed.
S3: after collecting is completed, the filter tape pressing device 2 is lifted up, and collecting counting is performed by the detector 3.
S4: after the counting is completed, new test filter tape is moved in place by using the filter tape feed device 1, and the next round of collecting is performed.

Specifically, when the monitor starts work, first, the main control panel 7 controls the filter tape pressing drive motor 74 to work through the relay 71. The output shaft of the filter tape pressing drive motor 74 rotates to drive the cam 742 to rotate. The convex edge of the cam 742 is in contact with the head pressing disc 23 to jack the head pressing disc 23 up, so that the head pressing block 21 is lifted up. When the first limit switch 52 returns a signal to the main control panel 7, it indicates that the head pressing block 21 is lifted up in place, and then, the main control panel 7 controls the filter tape drive motor 72 to drive the primary filter tape belt pulley 11 to rotate a certain angle to feed filter tape, and the filter tape to be tested drives the limit wheels 13 to rotate. When a second limit switch 62 returns a signal to the main control panel 7, it indicates that the filter tape to be tested is in place, and then, the main control panel 7 controls the detector 3 to perform initial counting, and data of blank filter tape to be tested is recorded and is sent to the main control panel 7 for storing. Then, the main control panel 7 controls the filter tape pressing drive motor 74 to work. The convex edge of the cam 742 leaves the head pressing disc 23. The head pressing disc 23 moves downward under the action of the spring to drive the head pressing block 21 to move downward to press the filter tape to be tested. The first limit switch 52 returns a signal to the main control panel 7. The main control panel 7 controls the gas pump 83 to work after receiving the signal. The gas to be tested is pumped, and gas to be tested passes through the head pressing block 21 and passes through the filter tape to be tested. Particulate matters in the ambient dust therein remain on the filter tape to be tested. Through a certain time of collecting, the main control panel 7 controls the gas pump 83 to stop working. The filter tape pressing drive motor 74 works, the head pressing disc 23 drives the head pressing block 21 to be lifted up by rotating the cam 742. Then, the beam emitted by the β-ray emitter is received by the detector 33 after passing through the filter tape to be tested. The detector 33 sends collected results to the main control panel 7. The main control panel 7 performs a logarithmic operation on the received data and initial data in combination with the temperature and humidity data collected by the temperature and humidity collector 90. Finally, the concentration of the particulate matters in the atmosphere is obtained. After this round of collecting is completed, the main control panel 7 controls the filter tape drive motor 72 to work to move away the used filter tape to be tested, and then a round of collecting and detecting is performed.

## Claims

1. A portable on-line in-situ β-ray monitor, comprising a detection mechanism and a control mechanism electrically connected to each other, wherein the detection mechanism comprises a filter tape feed device (1), a filter tape pressing device (2), a detector (3), and a gas guide device (4); the gas guide device (4) is arranged in the filter tape pressing device (2) in a penetrating manner to guide a gas to be tested on the test filter tape; the filter tape pressing device (2) is arranged right above the detector (3) and is configured for pressing the test filter tape; the detector (3) is configured for counting; the control mechanism comprises a first detector (5) and a second detector (6); the filter tape pressing device (2) is connected to the first detector (5); and the filter tape feed device (1) is connected to the second detector (6), the filter tape feed device (1) is configured for storing and moving test filter tape; the first detector (5) is configured for detecting a down-pressing situation of the filter tape pressing device (2) and the second detector (6) is configured for detecting a filter tape feeding in-place situation;
the monitor being **characterized in that**:
the filter tape pressing device (2) comprises a head pressing block (21) and a filter tape pressing holder (22); a head pressing disc (23) is fixedly arranged outside the head pressing block (21) in a sleeving manner; and an upper end of the head pressing disc (23) is fixedly connected to the filter tape pressing holder (22) through a spring; and
a lower end of the head pressing block (21) is connected to a head pressing membrane (24); an upper end of the pressing head block (21) is connected to a head pressing gas pipe (25); and the bottom of the pressing head gas pipe (25) is fixed to an upper end of the filter tape pressing holder (22) through a head pressing seat.

2. The portable on-line in-situ β-ray monitor according to claim 1, wherein a gas guide chamber (26) is formed in the head pressing block (21); an end cover (27) is fixed to the bottom of the head pressing block (21) and forms a gas outlet (28) at the bottom of the head pressing block (21); the gas guide device (4) is fixed to the inner side of the end cover (27); and the gas guide device (4) and the gas outlet (28) are arranged coaxially.

3. The portable on-line in-situ β-ray monitor according to claim 2, wherein the detector (3) comprises a detecting frame(31); a head pressing plate (29) is fixed to the top of the detecting frame (31); a detecting hole (32) is formed at a position where the head pressing plate (29) is fixedly connected to the detecting frame (31),; and a detector (33) is fixed coaxially to a lower end of the detecting hole (32).

4. The portable on-line in-situ β-ray monitor according to claim 2, wherein the control mechanism comprises a main control panel (7) configured to realize overall control of the monitor; the main control panel (7) is connected to a drive group through a relay (71) and the main control panel (7) controls the gas pump (83) to work through the relay (71); the drive group comprises a filter tape drive motor (72), a flow rate control motor (73), and a filter tape pressing drive motor (74); and the filter tape feed device (1) comprises a primary filter tape belt pulley (11), a secondary filter tape belt pulley (12), and two limiting wheels (13), the primary filter tape belt pulley (11) is connected to a filter tape drive motor (72); and the filter tape drive motor (74) drives the primary filter tape belt pulley (11) to rotate to feed filter tape; the flow rate control motor (73) controls the overall gas inlet amount of the monitor, and the main control panel (11) controls the flow rate control motor (73) to work through the relay (71) so as to drive a regulating valve (82) to work; and the filter tape pressing drive motor (74) is fixed to a mounting plate (100) through a filter tape pressing motor frame (741) and the filter tape pressing drive motor (74) works so that the pressing block (21) of the filter tape pressing device (2) performs an operation of pressing the filter tape or lifting up the filter tape; an output shaft of the filter tape pressing drive motor (74) is connected to a cam (742); a side of the cam (742) is connected to the head pressing disc (23); and the cam (742) rotates under the driving of the filter tape pressing drive motor (74).

5. The portable on-line in-situ β-ray monitor according to claim 4, wherein the output shaft of the filter tape pressing drive motor (74) is also fixedly connected to the cam (742) through the first detector (5).

6. The portable on-line in-situ β-ray monitor according to claim 3, wherein a mounting plate (100) is fixed between the detecting mechanism and the control mechanism; a connecting hole (101) is formed in the position, corresponding to a gas outlet of the detecting frame (31), of the mounting plate (100); the connecting hole (101) is connected to a gas guide pipe; the other end of the gas guide pipe is connected to a flow regulating device; the flow regulating device comprises a flowmeter (81); and the flowmeter (81) is connected between the connecting hole (101) and a regulating valve (82) through the gas guide pipe, and the other end of the regulating valve (82) is connected to a gas pump (83).

7. The portable on-line in-situ β-ray monitor according to claim 6, wherein the mounting plate (100) is fixedly arranged in the middle of a box body; and a collecting mechanism is fixedly arranged on the box body.

8. A monitoring method of the portable on-line in-situ β-ray monitor, the monitoring method being implemented by the portable on-line in-situ β-ray monitor according to any one of claims 1 to 7, comprising the following steps:
S1: lifting up the filter tape pressing device (2), moving test filter tape in place by the filter tape feed device (1), and performing initial counting by the detector (3);
S2: after the counting is completed, pressing down the filter tape pressing device (2), introducing a gas to be tested through the gas guide device (4), and performing collecting;
S3: after collecting is completed, lifting up the filter tape pressing device (2), and performing collecting counting by the detector (3); and
S4: after the counting is completed, moving new test filter tape in place by using the filter tape feed device (1), and performing next round of collecting.

## Patentansprüche

1. Tragbarer online-in-situ-β-Strahlmonitor, der einen Erfassungsmechanismus und einen Steuermechanismus umfasst, die elektrisch miteinander verbunden sind, wobei der Erfassungsmechanismus eine Filterbandzufuhrvorrichtung (1), eine Filterbandpressvorrichtung (2), einen Detektor (3) und eine Gasführungsvorrichtung (4) umfasst; die Gasführungsvorrichtung (4) in der Filterbandpressvorrichtung (2) durchdringend ausgelegt ist, um ein zu prüfendes Gas auf das Testfilterband zu führen; die Filterbandpressvorrichtung (2) direkt über dem Detektor (3) angeordnet und zum Pressen des Testfilterbandes ausgelegt ist; der Detektor (3) zum Zählen konfiguriert ist; der Kontrollmechanismus einen ersten Detektor (5) und einen zweiten Detektor (6) umfasst; die Filterbandpressvorrichtung (2) mit dem ersten Detektor (5) verbunden ist; und die Filterbandzufuhrvorrichtung (1) mit dem zweiten Detektor (6) verbunden ist, die Filterbandzufuhrvorrichtung (1) zum Speichern und Bewegen von Testfilterband konfiguriert ist; der erste Detektor (5) dafür konfiguriert ist, eine Situation des Herunterdrückens der Filterbandpressvorrichtung (2) zu erfassen, und der zweite Detektor (6) dafür konfiguriert ist, eine Situation der Zuführung des Filterbandes an Ort und Stelle zu erfassen;
wobei der Monitor **dadurch gekennzeichnet ist, dass**:
die Filterbandpressvorrichtung (2) einen Kopfpressblock (21) und einen Filterbandpresshalter (22) umfasst; eine Kopfpressscheibe (23) fest außerhalb des Kopfpressblocks (21) in einer hülsenartigen Weise ausgelegt ist; und ein oberes Ende der Kopfpressscheibe (23) fest mit dem Filterbandpresshalter (22) durch eine Feder verbunden ist; und
ein unteres Ende des Kopfpressblocks (21) mit einer Kopfpressmembran (24) verbunden ist; ein oberes Ende des Kopfpressblocks (21) mit einem Kopfpressgasrohr (25) verbunden ist; und das untere Ende des Kopfpressgasrohrs (25) mit einem oberen Ende des Filterbandpresshalters (22) durch einen Kopfpresssitz verbunden ist.

2. Tragbarer online-in-situ-β-Strahlmonitor nach Anspruch 1, wobei eine Gasleitkammer (26) in dem Kopfpressblock (21) gebildet ist; eine Endabdeckung (27) an der Unterseite des Kopfpressblocks (21) befestigt ist und einen Gasauslass (28) an der Unterseite des Kopfpressblocks (21) bildet; die Gasführungsvorrichtung (4) an der Innenseite der Endabdeckung (27) befestigt ist; und die Gasführungsvorrichtung (4) und der Gasauslass (28) koaxial ausgelegt sind.

3. Tragbarer online-in-situ-β-Strahlmonitor nach Anspruch 2, wobei der Detektor (3) einen Erfassungsrahmen (31) umfasst; eine Kopfdruckplatte (29) an der Oberseite des Erfassungsrahmens (31) befestigt ist; ein Erfassungsloch (32) an einer Position gebildet ist, an der die Kopfpressplatte (29) fest mit dem Erfassungsrahmen (31) verbunden ist; und ein Detektor (33) koaxial zu einem unteren Ende des Erfassungslochs (32) befestigt ist.

4. Tragbarer online-in-situ-β-Strahlmonitor nach Anspruch 2, wobei der Steuermechanismus ein Hauptbedienfeld (7) umfasst, das dafür konfiguriert ist, die Gesamtsteuerung des Monitors zu realisieren; das Hauptbedienfeld (7) über ein Relais (71) mit einer Antriebsgruppe verbunden ist und das Hauptbedienfeld (7) die Gaspumpe (83) steuert, um über das Relais (71) zu arbeiten; die Antriebsgruppe einen Filterbandantriebsmotor (72), einen Durchflussmengensteuermotor (73) und einen Filterbandpressantriebsmotor (74) umfasst; und die Filterbandzufuhrvorrichtung (1) eine primäre Filterbandriemenscheibe (11), eine sekundäre Filterbandriemenscheibe (12) und zwei Begrenzungsräder (13) umfasst, wobei die primäre Filterbandriemenscheibe (11) mit einem Filterbandantriebsmotor (72) verbunden ist; und der Filterbandantriebsmotor (74) die primäre Filterbandriemenscheibe (11) antreibt, um sich zu drehen, um das Zufuhrfilterband zu drehen; der Durchflussmengensteuermotor (73) die Gesamtgaseinlassmenge des Monitors steuert, und das Hauptsteuerpult (11) den Durchflussmengensteuermotor (73) steuert, um über das Relais (71) zu arbeiten, um ein Regelventil (82) zu betreiben; und der Filterbandpressantriebsmotor (74) durch einen Rahmen (741) für den Motor zum Pressen des Filterbandes an einer Montageplatte (100) befestigt ist, und der Filterbandpressantriebsmotor (74) so arbeitet, dass der Pressblock (21) der Filterbandpressvorrichtung (2) eine Operation zum Pressen des Filterbandes oder zum Anheben des Filterbandes durchführt; eine Ausgangswelle des Filterbandpressantriebsmotors (74) mit einem Nocken (742) verbunden ist; eine Seite des Nockens (742) mit der Kopfpressscheibe (23) verbunden ist; und sich der Nocken (742) unter dem Antrieb des Filterbandpressantriebsmotors (74) dreht.

5. Tragbarer online-in-situ-β-Strahlmonitor nach Anspruch 4, wobei die Ausgangswelle des Filterbandantriebsmotors (74) über den ersten Detektor (5) auch fest mit der Nocke (742) verbunden ist.

6. Tragbarer online-in-situ-β-Strahlmonitor nach Anspruch 3, wobei eine Montageplatte (100) zwischen dem Erfassungsmechanismus und dem Steuermechanismus befestigt ist; ein Verbindungsloch (101) in der Position, die einem Gasauslass des Erfassungsrahmens (31) entspricht, der Montageplatte (100) gebildet ist; das Verbindungsloch (101) mit einem Gasführungsrohr verbunden ist; das andere Ende des Gasführungsrohrs mit einer Durchflussregulierungsvorrichtung verbunden ist; die Durchflussregulierungsvorrichtung einen Durchflussmesser (81) umfasst; und der Durchflussmesser (81) zwischen der Verbindungsöffnung (101) und einem Regulierventil (82) durch das Gasführungsrohr verbunden ist, und das andere Ende des Regulierventils (82) mit einer Gaspumpe (83) verbunden ist.

7. Tragbarer online-in-situ-β-Strahlmonitor nach Anspruch 6, wobei die Montageplatte (100) fest in der Mitte eines Gehäusekörpers ausgelegt ist; und ein Auffangmechanismus fest auf dem Gehäusekörper ausgelegt ist.

8. Überwachungsverfahren des tragbaren online-in-situ-β-Strahlmonitors, wobei das Überwachungsverfahren durch den tragbaren online-in-situ-β-Strahlmonitor nach einem der Ansprüche 1 bis 7 implementiert wird und folgende Schritte umfasst:
S1: Anheben der Filterbandpressvorrichtung (2), Bewegen des Testfilterbandes durch die Filterbandzufuhrvorrichtung (1) und Durchführen einer ersten Zählung durch den Detektor (3);
S2: nach Beendigung des Zählens, Herunterdrücken der Filterbandpressvorrichtung (2), Einleiten eines zu prüfenden Gases durch die Gasführungsvorrichtung (4) und Durchführen des Auffangens;
S3: nach Beendigung des Sammelns, Anheben der Filterbandpressvorrichtung (2) und Durchführen der Sammelzählung durch den Detektor (3); und
S4: nach Beendigung des Zählens, Einlegen eines neuen Testfilterbandes mit Hilfe der Filterbandzufuhrvorrichtung (1) und Durchführen der nächsten Sammelrunde.

## Revendications

1. Dispositif portable de surveillance de rayons bêta in situ en ligne, comportant un mécanisme de détection et un mécanisme de commande reliés électriquement l'un à l'autre, dans lequel le mécanisme de détection comporte un dispositif d'alimentation de bande filtrante (1), un dispositif de compression de bande filtrante (2), un détecteur (3) et un dispositif de guidage de gaz (4) ; le dispositif de guidage de gaz (4) est agencé de manière pénétrante dans le dispositif de compression de bande filtrante (2) pour guider un gaz devant être testé sur la bande filtrante d'essai ; le dispositif de compression de bande filtrante (2) est agencé juste au-dessus du détecteur (3) et est configuré pour comprimer la bande filtrante d'essai ; le détecteur (3) est configuré à des fins de comptage ; le mécanisme de commande comporte un premier détecteur (5) et un deuxième détecteur (6) ; le dispositif de compression de bande filtrante (2) est relié au premier détecteur (5) ; et le dispositif d'alimentation de bande filtrante (1) est relié au deuxième détecteur (6), le dispositif d'alimentation de bande filtrante (1) est configuré pour stocker et déplacer la bande filtrante d'essai ; le premier détecteur (5) est configuré pour détecter une situation de compression vers le bas du dispositif de compression de bande filtrante (2) et le deuxième détecteur (6) est configuré pour détecter une situation d'alimentation de bande filtrante sur place ;
le dispositif de surveillance étant **caractérisé en ce que** :
le dispositif de compression de bande filtrante (2) comporte un bloc de compression de tête (21) et un support de compression de bande filtrante (22) ; un disque de compression de tête (23) est agencé de manière fixe à l'extérieur du bloc de compression de tête (21) tout comme un manchonnage ; et une extrémité supérieure du disque de compression de tête (23) est reliée de manière fixe au support de compression de bande filtrante (22) par le biais d'un ressort ; et
une extrémité inférieure du bloc de compression de tête (21) est reliée à une membrane de compression de tête (24) ; une extrémité supérieure du bloc de compression de tête (21) est reliée à une conduite de gaz de compression de tête (25) ; et le fond du tuyau de gaz de tête de compression (25) est fixé sur une extrémité supérieure du support de compression de bande filtrante (22) par le biais d'un siège de compression de tête.

2. Dispositif portable de surveillance de rayons bêta in situ en ligne selon la revendication 1, dans lequel une chambre de guidage de gaz (26) est formée dans le bloc de compression de tête (21) ; un couvercle d'extrémité (27) est fixé sur le fond du bloc de compression de tête (21) et forme une sortie (28) au niveau du fond du bloc de compression de tête (21) ; le dispositif de guidage de gaz (4) est fixé sur le côté intérieur du couvercle d'extrémité (27) ; et le dispositif de guidage de gaz (4) et la sortie de gaz (28) sont agencés de manière coaxiale.

3. Dispositif portable de surveillance de rayons bêta in situ en ligne selon la revendication 2, dans lequel le détecteur (3) comporte un cadre de détection (31) ; une plaque de compression de tête (29) est fixée sur la partie supérieure du cadre de détection (31) ; un trou de détection (32) est formé au niveau d'une position où la plaque de compression de tête (29) est reliée de manière fixe au cadre de détection (31) ; et un détecteur (33) est fixé de manière coaxiale sur une extrémité inférieure du trou de détection (32).

4. Dispositif portable de surveillance de rayons bêta in situ en ligne selon la revendication 2, dans lequel le mécanisme de commande comporte un panneau de commande principal (7) configuré pour réaliser une commande globale du dispositif de surveillance ; le panneau de commande principal (7) est relié à un groupe d'entraînement par le biais d'un relais (71) et le panneau de commande principal (7) commande la pompe à gaz (83) pour qu'elle fonctionne par le biais du relais (71) ; le groupe d'entraînement comporte un moteur d'entraînement de bande filtrante (72), un moteur de commande de débit d'écoulement (73) et un moteur d'entraînement de compression de bande filtrante (74) ; et le dispositif d'alimentation de bande filtrante (1) comporte une poulie de courroie de bande filtrante primaire (11), une poulie de courroie de bande filtrante secondaire (12) et deux roues de limitation (13), la poulie de courroie de bande filtrante primaire (11) est reliée à un moteur d'entraînement de bande filtrante (72), et le moteur d'entraînement de bande filtrante (74) entraîne la poulie de courroie de bande filtrante primaire (11) à des fins de rotation pour alimenter la bande filtrante ; le moteur de commande de débit d'écoulement (73) commande la quantité totale d'entrée de gaz du dispositif de surveillance, et le panneau de commande principal (11) commande le moteur de commande de débit d'écoulement (73) pour qu'il fonctionne par le biais du relais (71) de manière à entraîner une soupape de régulation (82) à des fins de fonctionnement ; et le moteur d'entraînement de compression de bande filtrante (74) est fixé sur une plaque de montage (100) par le biais d'un châssis (741) de moteur de compression de bande filtrante et le moteur d'entraînement de compression de bande filtrante (74) fonctionne de telle sorte que le bloc de compression (21) du dispositif de compression de bande filtrante (2) effectue une opération de compression sur la bande filtrante ou de levage de la bande filtrante ; un arbre de sortie du moteur d'entraînement de compression de bande filtrante (74) est relié à une came (742) ; un côté de la came (742) est relié au disque de compression de tête (23) ; et la came (742) tourne sous l'effet de l'entraînement du moteur d'entraînement de compression de bande filtrante (74).

5. Dispositif portable de surveillance de rayons bêta in situ en ligne selon la revendication 4, dans lequel l'arbre de sortie du moteur d'entraînement de compression de bande filtrante (74) est également relié de manière fixe à la came (742) par le biais du premier détecteur (5).

6. Dispositif portable de surveillance de rayons bêta in situ en ligne selon la revendication 3, dans lequel une plaque de montage (100) est fixée entre le mécanisme de détection et le mécanisme de commande ; un trou de liaison (101) est formé dans la position, qui correspondant à une sortie de gaz du cadre de détection (31), de la plaque de montage (100) ; le trou de liaison (101) est relié à un tuyau de guidage de gaz ; l'autre extrémité du tuyau de guidage de gaz est reliée à un dispositif de régulation d'écoulement ; le dispositif de régulation d'écoulement comporte un débitmètre (81) ; et le débitmètre (81) est relié entre le trou de liaison (101) et une soupape de régulation (82) par le biais du tuyau de guidage de gaz, et l'autre extrémité de la soupape de régulation (82) est reliée à une pompe à gaz (83).

7. Dispositif portable de surveillance de rayons bêta in situ en ligne selon la revendication 6, dans lequel la plaque de montage (100) est agencée de manière fixe au milieu d'un corps de caisson ; et un mécanisme de collecte est agencé de manière fixe sur le corps de caisson.

8. Procédé de surveillance du dispositif portable de surveillance de rayons bêta in situ en ligne, le procédé de surveillance étant mis en œuvre par le dispositif portable de surveillance de rayons bêta in situ en ligne selon l'une quelconque des revendications 1 à 7, comportant les étapes suivantes consistant à :
S1 : soulever le dispositif de compression de bande filtrante (2), déplacer la bande filtrante d'essai en place par le dispositif d'alimentation de bande filtrante (1), et effectuer un décompte initial par le détecteur (3) ;
S2 : une fois le décompte terminé, appuyer sur le dispositif de compression de bande filtrante (2), introduire un gaz devant être testé à travers le dispositif de guidage de gaz (4) et effectuer la collecte ;
S3 : une fois la collecte terminée, soulever le dispositif de compression de bande filtrante (2) et effectuer le décompte de collecte par le détecteur (3) ; et
S4 : une fois le décompte terminé, déplacer la nouvelle bande filtrante d'essai en place à l'aide du dispositif d'alimentation de bande filtrante (1), et effectuer la prochaine opération de collecte.
